# EUROPÄISCHE PATENTSCHRIFT

(11) **EP 3 771 905 B1**
(45) Veröffentlichungstag und Bekanntmachung des Hinweises auf die Patenterteilung: **02.08.2023**
(21) Anmeldenummer: 20183707.7
(22) Anmeldetag: 02.07.2020
(51) Int. Cl.: G01N 27/02, B01D 53/04, F25B 49/00, G01N 25/48, G01N 33/00

(54) **MESSFORMKÖRPER ZUR MESSUNG DES BELADUNGSZUSTANDS BEI EINER ADSORPTION UND ZUGEHÖRIGES MESSVERFAHREN**
MEASURING BODY FOR MEASURING THE ADSORPTION STATE AND CORRESPONDING MEASURING METHOD
ORGANE DE MESURE POUR MESURER L'ÉTAT D'ADSORPTION ET MÉTHODE DE MESURE CORRESPONDANTE

(30) Priorität: 01.08.2019 DE 102019120848
(43) Veröffentlichungstag der Anmeldung: 03.02.2021
(73) Patentinhaber: Vaillant GmbH, 42859 Remscheid (DE)
(72) Erfinder: Lingk, Tobias, 42799 Leichlingen (DE); Krampe-Zadler, Christof, 44628 Herne (DE); Spahn, Hans-Josef, 40699 Erkrath (DE)
(74) Vertreter: Popp, Carsten

(56) Entgegenhaltungen:
- DE-A1- 1 523 021
- DE-A1- 2 609 869
- DE-A1-102010 003 710
- DE-A1-102017 126 952
- Kraft ET AL: "Determination of Load Dependent Thermal Conductivity of Porous Adsorbents", Proceddings of the COMSOL Conference, 1. Januar 2016 (2016-01-01), Seiten 1-7, XP055720168, Gefunden im Internet: URL:https://www.comsol.se/paper/download/3 56771/kraft_paper.pdf [gefunden am 2020-08-04]
- JAEGLE M ET AL: "Thermal-electrical Impedance Spectroscopy for Fluid Characterisation", PROCEDIA ENGINEERING, ELSEVIER BV, NL, Bd. 168, 4. Januar 2017 (2017-01-04), Seiten 770-773, XP029874768, ISSN: 1877-7058, DOI: 10.1016/J.PROENG.2016.11.276

## Beschreibung

Die Erfindung betrifft die Messung der adsorptiven Beladung von Aktivkohle mit gasförmigen Kohlenwasserstoffen und anderen organischen Stoffen Das Adsorbent dient beispielsweise zur Adsorption von R290 oder sonstigen Arbeitsfluiden, die Alkane und Alkene enthalten und in Kältekreisen zum Einsatz kommen, sowie einer Vielzahl von anderen industriellen Einsatzzwecken.

Aktivkohle ist seit langem bekannt als Abscheidemittel für Verunreinigungen in Gasen und Flüssigkeiten. Sie wird aus Kohle gewonnen und mittels Chemikalienbehandlung und überhitztem Wasserdampf aktiviert. Im Handel ist sie in verschiedenen Formen erhältlich, üblich sind Pulver, Pellets, Gewebe, Schüttungen, gesinterte Fest- und Formkörper und viele mehr, wobei in vielen Anwendungsfällen Trägerstrukturen verwendet werden. Die Abscheidewirkung wird erzielt durch eine sehr große innere Oberfläche sowie durch gezielt eingebrachte Imprägnierungen, die bestimmte Verunreinigungen binden.

Apparativ üblich sind durchströmte Kartuschen sowie Festbetten mit Rückhaltevorrichtungen, die Aktivkohle in den oben genannten Formen enthalten. Die Auslegung solcher Apparate erfolgt, indem man die Beladungskapazität der Aktivkohle für jeden der abzuscheidenden Stoffe bestimmt, wobei die Beladungskapazität in der Regel stark temperaturabhängig ist, und man die Durchströmungsmenge bestimmt. Hierbei ergibt sich im Idealfall ein Adsorptionsgleichgewicht und eine wandernde Durchbruchsfront zwischen beladenem und noch unbeladenem Adsorbens. Das gilt vor allem für Abscheidungsvorgänge aus der Gasphase.

Leider ist ein solcher Idealfall in der Praxis selten. So kennt man die Durchströmungsmenge des betreffenden Stoffes oft nicht, vor allem dann, wenn die Aktivkohle nur zur Sicherung dient und keine regelhafte Beladung erfolgen soll, oder wenn sich die Temperatur und damit die Beladungskapazität plötzlich stark ändern, was allein schon aufgrund der freiwerdenden Adsorpionswärme geschehen kann, oder wenn verschiedene abzuscheidende Stoffe sich gegenseitig verdrängen.

Praktisch muss man also bislang die Konzentration des abzuscheidenden Stoffes am Ausgang des Adsorbers messen, um zu wissen, ob oder wann der Adsorber beladen ist und man kann nicht einfach erkennen, wie weit der Ladezustand innerhalb des Adsorbers bereits fortgeschritten ist und welche Restkapazität noch vorhanden ist. Das ist insofern unbefriedigend, als man möglicherweise eine gewisse Vorlaufzeit für einen Filterwechsel benötigt und abschätzen möchte, wann der Filterwechsel erforderlich wird, und zwar bevor der abzuscheidende Stoff durchbricht.

Außerdem kann sich die Aktivkohle mit der Zeit verändern, weil andere Gasbestandteile hineindiffundieren, wodurch die Aktivkohle altert. Man kennt also den Beladungszustand und die verbliebene Aufnahmekapazität nicht, wenn die Aktivkohle nur zur Sicherheit dient und keine regelmäßigen oder zyklischen Beladungen erfolgen. Hinzu kommt, dass Aktivkohle eine Vielzahl verschiedener Substanzen abscheidet, die im Laufe einer einsetzenden Beladung mit besser adsorbierbaren Stoffen verdrängt werden und dabei desorbieren. Dasselbe gilt grundsätzlich auch für andere Adsorbenzien.

Die Aufgabe der Erfindung ist daher, eine Messvorrichtung bereitzustellen, mit der der Beladungszustand und die zeitliche Beladungszustandsänderung eines Adsorbens in einer Adsorbensschüttung oder Adsorbenspackung kontinuierlich oder diskontinuierlich festgestellt werden kann. Hierbei wird von der Impedanz und von Temperaturänderungen Gebrauch gemacht.

In der Veröffentlichung "Jürgen U. Keller, Rainer Staudt; Gas Adsorption Equilibria; Springer Science + Business Media, Boston 2010, Seiten 289 ff." werden eine Messvorrichtung und ein Verfahren skizziert, mit dem man derartige Beladungszustände mittels Impedanz- und Permittivitätsmessungen zwar grundsätzlich bestimmen kann, um etwa Adsorptionsgleichgewichte und Adsorptionswärmen, also Stoffwerte, zu ermitteln. Die dort vorgestellten Apparaturen und Auswertungsverfahren eignen sich aber weder für dynamische Messungen noch für den industriellen Serieneinsatz, sondern nur für stationäre Messungen zum Vergleich der Eignung verschiedener Adsorbenzien für unterschiedlichste Adsorbate und Gasmischungen. Die Zielsetzung und somit die technische Ausführung ist daher völlig verschieden. Eine direkte Anwendung scheidet daher aus.

Eine andere Messmethodik ist die thermische Impedanzmessung, bei der die thermische Leitfähigkeit einer Flüssigkeit oder eines Festkörpers mittels aufgebrachter thermischer Schwingungen gemessen wird. Hierzu muss man die Probe erhitzen und sie muss sich danach wieder auf ihren Ausgangswert abkühlen. Der Wärmeleitfähigkeit des durchlaufenen Materials entsprechend werden diese Wärmeschwingungen von einem Temperatursensor aufgefangen und ihre Phasenverschiebung der Schwingung wird gemessen. Mit Hilfe der 3w-Methode wird die thermische Impedanz ermittelt, die ebenfalls als Maß für die Beladung dienen kann, da sich die Wärmeleitung eines Reinstoffes von der eines beladenen Stoffes unterscheidet.

Ein solches thermoelektrisches Impedanzmessverfahren ist beispielsweise bekannt aus "M. Jaegle e.a., Thermal-electrical impedance spectroscopy for fluid characterization, AMA Conferences 2017 - SENSOR 2017 and IRS 2017". Das Aufbringen von Temperaturschwingungen ist bei beladenen Adsorbenzien jedoch insofern problematisch, als jede Temperaturerhöhung das Gleichgewicht der Adsorption beeinflusst. Erhöht man also an einer Stelle einer Adsorbensschüttung, beispielsweise einer Schüttung aus Aktivkohle, die Temperatur, dann desorbiert ein Anteil des Adsorbats und das Messergebnis wird verfälscht. Die Temperaturschwingungen müssen daher mit einer sehr kleinen Amplitude durchgeführt werden. Das schränkt ihre Anwendbarkeit in einer Aktivkohleschüttung, die aus unregelmäßig geformten Partikeln besteht, erheblich ein, denn die Temperaturerhöhung müsste zunächst auf die einzelnen Partikel übertragen werden, und diese müssten die Wärme bis zum Temperatursensor weiterleiten. Bewegungen der Partikel in der Schüttung aufgrund etwa von Vibrationen würden eine definierte Wärmeleitung erschweren oder unmöglich machen.

Das erhaltene Signal wird aber bei durchströmten Schüttungen auch durch die Konvektion der Strömung gestört, was vorliegend ein Nachteil ist, da die Durchströmung während der gewünschten Beladungsmessung in der Schüttung nicht genau genug bekannt ist, um sie rechnerisch angemessen berücksichtigen zu können und die Strömungsgeschwindigkeit selbst nur im freien durchströmten Querschnitt mittels Hitzdraht gemessen werden kann. Außerdem ist es nur schlecht möglich, verschiedene Adsorbate allein aufgrund ihrer thermischen Leitfähigkeit präzise voneinander zu unterscheiden, wenn ihre Beladungsdichten unterschiedlich sind.

Die DE 10 2010 003 710 A1 beschreibt eine Vorrichtung und ein Verfahren zur Bestimmung eines Anteils eines adsorbierten Stoffes, welcher in einen Formkörper, Granulat oder Pulver aus einem Zeolith, einer Zeolithverbindung oder Silikagel als Adsorbermaterial enthalten ist, mittels elektrischer Impedanz. Der Formkörper wird dabei mittels des CIM-Verfahrens hergestellt, die Elektroden sind im Formkörper eingebettet. Weitere Beschreibungen von Teilaspekten finden sich in "Kraft et. Al.: "Determination of Load Dependent Thermal Conductivity of Porous Adsorbents", Proceedings of the COMSOL Conference, 1. Januar 2016, Seiten 1-7, weiterhin in der DE 15 23 021 A1, der DE 26 09 869 A1 und der DE 10 2017 126952 A1.

Die Aufgabe der Erfindung ist daher, ein Messverfahren und eine Vorrichtung bereitzustellen, mit dem der Beladungszustand eines Adsorbens innerhalb einer Schüttung oder Packung bestimmt werden kann.

Die vorliegende Erfindung löst dieses Problem mithilfe einer Vorrichtung zur kombinierten Impedanzmessung und ist außer für Aktivkohle auch für jedes andere Adsorbens, welches wärmeleitend ist und als Dielektrikum wirkt, anwendbar, ferner auch innerhalb von Fluiden wie Flüssigkeiten oder Gasen, wobei nur die Verwendung von Aktivkohle als Adsorbens Gegenstand der vorliegenden Erfindung ist. Als Impedanz werden sowohl die elektrische Impedanz als auch die thermische Impedanz ermittelt.

Als Messvorrichtung dient ein Messformkörper mit einer Platine, die mit Sensoren, Widerständen und Leitern bestückt und mit Lack isoliert ist. Vorzugsweise wird eine SMD-Platine verwendet, bei der die Bestückungen nur aufgebracht werden und ein Durchlöten nicht vorgesehen ist. Die Platine ist von Adsorbensmaterial, im Fall der vorliegenden Erfindung Aktivkohle, umgeben, welches dem Material entspricht, welches im zu messenden Adsorber verwendet wird, jedoch pulverisiert und so agglomeriert ist, dass beladenes Gas wie durch die Adsorbenspartikel der Schüttung diffundieren kann. Auf diese Weise stellt sich in der die Messplatine umgebenden Aktivkohle, derselbe Beladungszustand wie in der ihn umgebenden Schüttung ein. Die Fixierung des Adsorbenspulvers auf der Platine kann durch Aufkleben oder durch Verbinden mit dem Isolierlack der Platine erfolgen

Der Messformkörper hat vorzugsweise die gleiche Größenordnung wie die Schüttungspartikel selbst. Die Abstände zwischen den Signalgebern und den Signalnehmern sind dabei so klein, dass auch mit sehr kleinen Energieeinträgen gemessen werden kann, was die oben beschriebenen Probleme verfälschender Desorption oder Gasströmungen gegenstandslos werden lässt.

Die einzelnen Messformkörper werden mittels üblicher Stromkabel mit elektrischer Energie versorgt und können an verschiedenen Stellen in einer Aktivkohleschüttung platziert werden. Mit ihnen wird sowohl eine thermische als auch eine elektrische Impedanzmessung über einen weiten Frequenzbereich durchgeführt. Dieser Frequenzbereich hängt jeweils vom Adsorbens und vom Adsorptiv ab.

Der Messformkörper enthält auf seiner Platine mindestens zwei gegenüber angeordnete Elektroden für die elektrische Impedanzmessung und mindestens einen Heizwiderstand mit mindestens einem gegenüberliegendem Thermosensor für die thermische Impedanzmessung. Zwischen den Elektroden und zwischen Heizwiderstand und Thermosensor ist Aktivkohle als fein gemahlenes Adsorbens fixiert. In Ausgestaltungen ist vorgesehen, dass die Elektroden der elektrischen Impedanzmessung als konzentrische Ringelektroden ausgeführt sind und bei der thermischen Impedanzmessung mehrere Ohm'sche Widerstände gleichmäßig in umschließender Weise um einen Thermosensor herum angeordnet sind. Die Widerstände sind dabei parallel geschaltet, die Anordnung verringert die Richtungsabhängigkeit der Wärmeleitung während der Durchströmung.

In weiteren Ausgestaltungen ist vorgesehen, dass die thermische Impedanzmessung und die elektrische Impedanzmessung nebeneinander auf derselben Platine angeordnet sind. In einer anderen Ausgestaltung ist vorgesehen, dass die beiden Impedanzmessungen auf verschiedenen Platinen stattfinden, die geometrisch so miteinander verbunden sind, dass sich zwischen ihnen gemahlenes Adsorbens sicher einschließen lässt. Diese Methode ergibt robustere Formmesskörper.

Die Erfindung löst die Aufgabe auch durch ein Messverfahren zur Messung des Beladungszustands eines Adsorbens vor oder während eines Adsorptionsvorgangs oder nach einem Adsorptionsvorgang unter Verwendung eines oder mehrerer Messformkörper. Hierbei wird zunächst eine Kalibrierung für den abzuscheidenden Stoff oder die Stoffmischung vorgenommen und nachfolgend kann das Messverfahren für Vergleichsmessungen eingesetzt werden.

Im ersten Schritt der Kalibrierung wird vorgesehen, dass
- zunächst an einer ersten Elektrode im unbeladenen Messformkörper eine hochfrequente Wechselspannung angelegt wird, deren Frequenz während der Messung in einem weiten Bereich durchfahren wird, an einer zweiten Elektrode, die der ersten Elektrode gegenüber angeordnet ist und wobei sich zwischen den beiden Elektroden Adsorbens findet, die Spannung erfasst wird, und dabei der Spannungsabfall und der Stromfluss zwischen den beiden Elektroden über diesen Frequenzbereich und daraus der Verlauf der elektrischen Impedanz über die Frequenz bestimmt werden,
- weiterhin die thermische Leitfähigkeit und die thermische Kapazität der Adsorbensschicht ermittelt werden, indem über einen weiten Frequenzbereich eine niederfrequente Temperaturschwingung mittels einer Heizvorrichtung aufgebracht und diese Schwingung mit einem Temperatursensor bestimmt und daraus die thermische Impedanz über die Frequenz bestimmt werden, wobei sich zischen der Heizvorrichtung und dem Temperatursensor Adsorbens befindet,
- in einem zweiten Schritt danach das Adsorbens kontrolliert mit einer der zu adsorbierenden Substanzen beladen wird und das Vorgehen des ersten Schrittes für das so beladene Adsorbens wiederholt wird,
- in weiteren Schritten danach eine schrittweise weitere Beladung wie im zweiten Schritt erfolgt, bis das Adsorbens vollständig beladen ist,
- durch Vergleich der gemessenen Impedanzen, der Wechselstrom-Widerstände, der thermischen Leitfähigkeit und der thermischen Kapazität ein Beladungskennfeld aus Wertepaaren von elektrischer und thermischer Impedanz für dieses Paar aus Adsorbens und Adsorptiv erstellt wird,
- diese Beladungskennlinie einer rechnergestützten Auswerteeinheit zugeführt wird.

In weiteren Schritten der Kalibrierung wird vorgesehen, dass
- die vorangegangenen Schritte für alle weiteren in Betracht kommenden Adsorptive einzeln vorgenommen werden, wobei der Messformkörper jedes Mal zuvor entladen wird,
- die erhaltenen Beladungskennlinien als ein Beladungskennfeld zusammengeführt werden,
- dieses Beladungskennfeld einer rechnergestützten Auswerteeinheit zugeführt wird.

Bei gleichzeitiger Beladung mit mehreren der im Beladungskennfeld gespeicherten Adsorptive wird in der Auswerteeinheit das Superpositionsprinzip angewendet. Auf diese Weise lassen sich lokale Beladungszustände messen und Verdrängungseffekte wie auch Durchbruchskurven im Voraus abschätzen.

In einer weiteren Ausgestaltung der Kalibrierung wird vorgesehen, dass die oben beschriebenen Schritte für verschiedene Temperaturen durchgeführt werden.

Das aus der Kalibrierung erhaltene Beladungskennfeld kann dann für alle baugleichen Apparate, Adsorbenzien, Adsorbate und Adsorptive verwendet werden, wobei es weder auf die Konzentrationen der Adsorptive in der jeweiligen Fluidphase noch auf die Umströmungsgeschwindigkeit ankommt. Das Messverfahren kann sowohl in der Gasphase als auch in der Flüssigphase angewendet werden.

In einer Ausgestaltung ist vorgesehen, dass einer oder mehrere Messformkörper in einer Schüttung oder einer Packung aus Adsorbensmaterial platziert werden, und dass in kurzen Abständen die Impedanzmessungen über die Frequenzbereiche durchgeführt werden. Hierbei werden bevorzugt die Frequenzbereiche durchfahren, die sich für die betreffenden Wertepaare während der vorangegangenen Kalibrierung als besonders selektiv erwiesen haben.

Die Erfindung wird nachfolgend anhand von 6 Prinzipskizzen näher erläutert. Hierbei zeigen:
- Fig. 1:: eine erste Ausführungsvariante der Messelektroden,
- Fig. 2:: eine zweite Ausführungsvariante der Messelektroden,
- Fig. 3:: eine Anordnung für die thermische Impedanzmessung,
- Fig. 4:: eine planare Anordnung der Impedanzmessungen auf einer SMD-Platine,
- Fig. 5:: eine integrierte zweistöckige Anordnung der Impedanzmessungen auf zwei SMD-Platinen,
- Fig. 6:: eine integrierte Anordnung mit umgebendem Adsorbens.

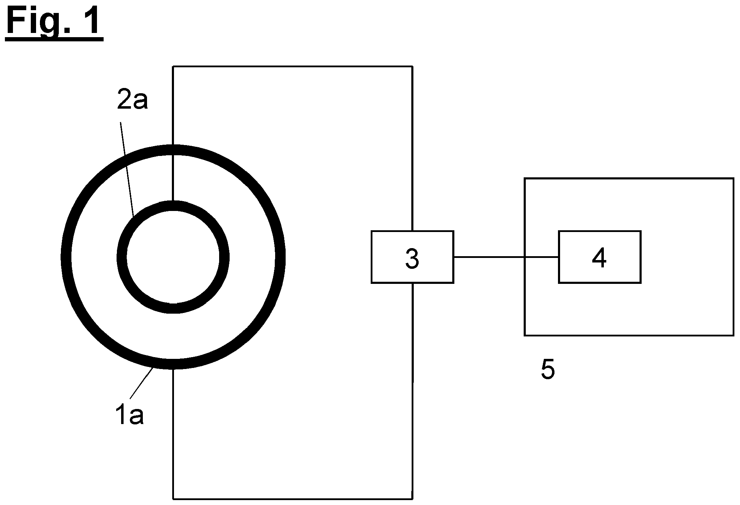

Fig. 1 zeigt eine erste Ausführungsvariante der Messelektroden mit konzentrisch angeordneten Ringelektroden 1a und 2a, die an einen Oszillator 3 angeschlossen sind. Dieser ist mit einem Frequenzzähler 4 verbunden, der die ermittelten Daten an eine MCU (Micro Controller Unit) 5 weiterleitet.

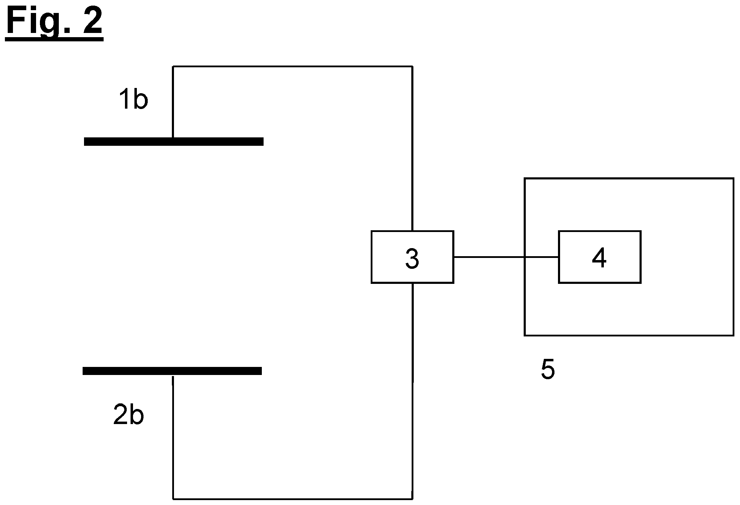

Fig. 2 zeigt eine zweite Ausführungsvariante der Messelektroden mit parallel zueinander angeordneten Elektroden 1b und 2b, die an einen Oszillator 3 angeschlossen sind. Dieser ist mit einem Frequenzzähler 4 verbunden, der die ermittelten Daten an eine MCU (Micro Controller Unit) 5 weiterleitet.

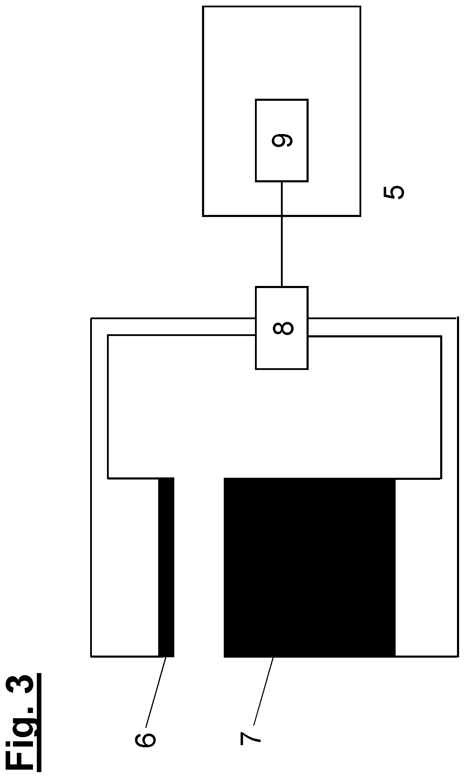

Fig. 3 zeigt eine Anordnung für die thermische Impedanzmessung mit einem Ohm'schen Gleichstromwiderstand, der das Heizelement 6 bildet, und einem Thermosensor 7, der die Temperaturschwankungen misst. Beide sind verbunden mit der Gleichstromquelle 8, die für den Gleichstromwiderstand 6 einen Gleichstrom mit einem sinusförmigen Spannungsverlauf entsprechend einer vorgegebenen Frequenz erzeugt. Dieser ist mit einem Frequenzzähler 9 verbunden, der die ermittelten Daten an eine MCU (Micro Controller Unit) 5 weiterleitet.

Während der in den Fig. 1 und 2 gezeigte Frequenzzähler 4 auf sehr hohe Schwingungsfrequenzen im Gigahertzbereich ausgelegt ist, ist der Frequenzzähler 9 auf sehr niedrige Schwingungsfrequenzen im Hertz- und Millihertzbereich ausgelegt. Die genaue Auslegung erfolgt entsprechend dem gewählten Adsorbens Aktivkohle und Adsorptiven bzw. Adsorbaten, die unterschiedliche hohe Frequenzbereiche erfordern.

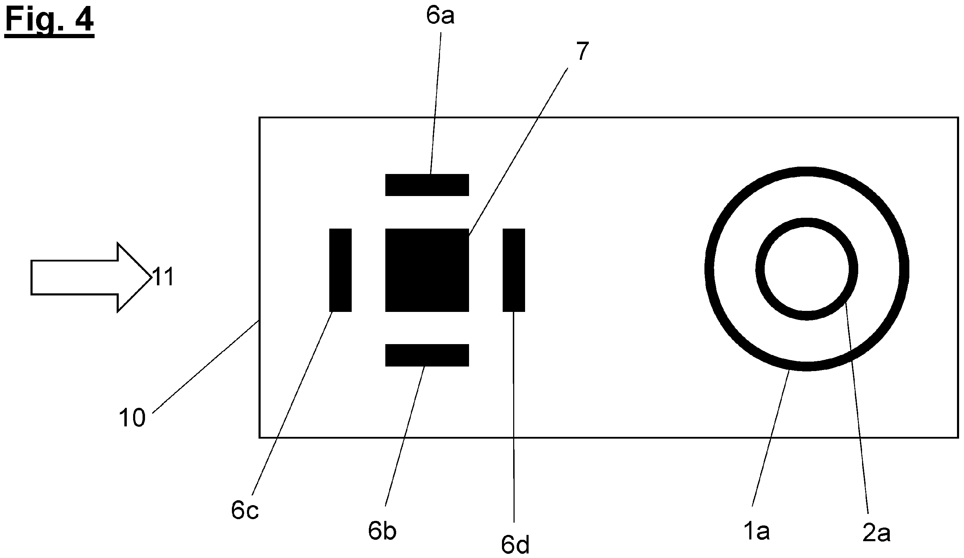

Fig. 4 zeigt eine planare Anordnung der Impedanzmessungen auf einer SMD-Platine 10, wobei auf der linken Seite die thermische Impedanz und auf der rechten Seite die elektrische Impedanz gemessen werden. Das mit Adsorptiv beladene Fluid gelangt aus der Richtung 11 auf die Platine und streicht darüber hinweg, wobei die SMD-Platine 10 und die Zwischenräume zwischen den Bestückungen vollständig mit Aktivkohleadsorbenspulver gefüllt sind. Dadurch, dass die Heizelemente 6a, 6b, 6c und 6d den Thermosensor 7 auf vier Seiten umschließen, ist die Messung der thermischen Impedanz ebenso unabhängig von der Strömungsrichtung 11 wie die Messung der elektrischen Impedanz mittels der konzentrischen Ringelektroden 1a und 2a.

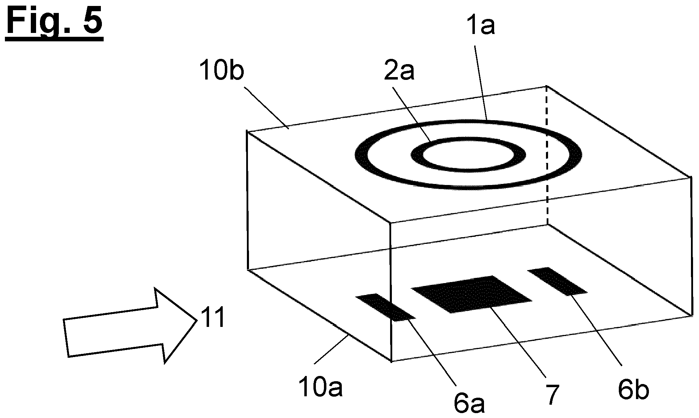

Fig. 5 zeigt eine integrierte zweistöckige Anordnung der Impedanzmessungen auf zwei SMD-Platinen 10a und 10b, die den Vorteil hat, dass das gemahlene Aktivkohleadsorbenspulver zwischen den beiden Platinen gut festgehalten wird, was eine sehr robuste Ausführung ist.

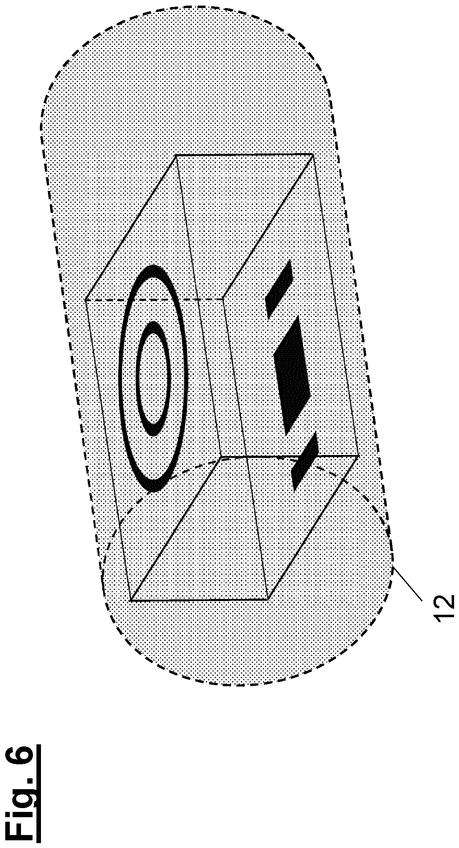

Fig. 6 zeigt eine integrierte Anordnung wie in Fig. 5 mit umgebendem Aktivkohleadsorbens 12, das in diesem Beispiel die Form von zylindrischen Pellets hat.

### Bezugszeichenliste

- 1a, 1b: Elektrode
- 2a, 2b: Elektrode
- 3: Oszillator
- 4: Frequenzzähler
- 5: MCU
- 6, 6a, 6b, 6c, 6d: Heizelement
- 7: Thermosensor
- 8: Gleichstromquelle
- 9: Frequenzzähler
- 10, 10a, 10b: SMD-Platine
- 11: Anströmrichtung
- 12: Aktivkohleadsorbenspulver

## Patentansprüche

1. Messformkörper zur Messung des Beladungszustands bei einer Adsorption, aufweisend mindestens eine Platine (10) mit Einrichtungen zur Messung der elektrischen und thermischen Impedanz, aufweisend mindestens 2 Elektroden (1, 2), die geeignet sind, den Strom einer hochfrequenten Wechselspannung zu übertragen, sowie mindestens einen Gleichstrom-Heizwiderstand (6) und einen Temperatursensor (7), ferner Leitungen für elektrische Anschlüsse
**dadurch gekennzeichnet, dass**
- die mindestens eine Platine (10) mit einem aufgebrachten Pulver aus Aktivkohleadsorbens (12) umhüllt ist, wobei dieses Aktivkohleadsorbens sowohl zwischen den Elektroden (1, 2) für hochfrequenten Wechselstrom als auch zwischen Heizelement (6) und Temperatursensor (7) angeordnet ist, und
- das Aktivkohleadsorbenspulver (12) in poröser Form agglomeriert auf der mindestens einen Platine (10) fixiert ist.

2. Messformkörper nach Anspruch 1, **dadurch gekennzeichnet, dass** eine planare SMD-Platine (10) verwendet wird, bei der die Messeinrichtungen der elektrischen und der thermischen Impedanz nebeneinander angeordnet sind.

3. Messformkörper nach Anspruch 1, **dadurch gekennzeichnet, dass** zwei SMD-Platinen (10a, 10b) verwendet werden, bei der die Messeinrichtungen der elektrischen und der thermischen Impedanz auf je einer Platine übereinander angeordnet sind.

4. Messformkörper nach einem der Ansprüche 1 bis 3, **dadurch gekennzeichnet, dass** für die Messung der thermischen Impedanz zwei gegenüberliegende Ohm'sche Widerstände als Heizelemente (6a, 6b) verwendet werden und ein Temperatursensor (7) zwischen diesen beiden Heizelementen (6a, 6b) angeordnet ist.

5. Messformkörper nach Anspruch 4, **dadurch gekennzeichnet, dass** für die Messung der thermischen Impedanz vier Ohm'sche Widerstände als Heizelemente (6a, 6b, 6c, 6d) verwendet werden, von denen je zwei (6a+6b, 6c+6d) einander gegenüberliegen, und ein Temperatursensor (7) zwischen diesen vier Heizelementen (6a, 6b, 6c, 6d) angeordnet ist.

6. Messformkörper nach einem der Ansprüche 1 bis 3, **dadurch gekennzeichnet, dass** für die Messung der elektrischen Impedanz zwei gegenüberliegende Flachelektroden (1b, 2b) verwendet werden.

7. Messformkörper nach einem der Ansprüche 1 bis 3, **dadurch gekennzeichnet, dass** für die Messung der elektrischen Impedanz zwei konzentrische Ringelektroden (1a, 2a) verwendet werden.

8. Messformkörper nach einem der Ansprüche 1 bis 7, **dadurch gekennzeichnet, dass** wenigstens eine der Abmessungen des Messformkörpers der eines Aktivkohleadsorbenspartikels entspricht.

9. Messformkörper nach Anspruch 8, **dadurch gekennzeichnet, dass** Form und Abmessungen des Messformkörpers der eines Aktivkohleadsorbenspartikels entspricht.

10. Messverfahren unter Verwendung eines Messformkörpers gemäß einem der vorangegangenen Ansprüche, **dadurch gekennzeichnet, dass** vor der Messung eine Kalibrierung zur Ermittlung eines Messkennfeldes durchgeführt wird.

11. Messverfahren nach Anspruch 10, **dadurch gekennzeichnet, dass** die Kalibrierung die folgenden Schritte umfasst, indem
- zunächst an einer ersten Elektrode im unbeladenen Messformkörper eine hochfrequente Wechselspannung angelegt wird, deren Frequenz während der Messung in einem weiten Bereich durchfahren wird, an einer zweiten Elektrode, die der ersten Elektrode gegenüber angeordnet ist und wobei sich zwischen den beiden Elektroden Adsorbens findet, die Spannung erfasst wird, und dabei der Spannungsabfall und der Stromfluss zwischen den beiden Elektroden über diesen Frequenzbereich und daraus der Verlauf der elektrischen Impedanz über die Frequenz bestimmt werden,
- weiterhin die thermische Leitfähigkeit und die thermische Kapazität der Adsorbensschicht ermittelt werden, indem über einen weiten Frequenzbereich eine niederfrequente Temperaturschwingung mittels einer Heizvorrichtung aufgebracht und diese Schwingung mit einem Temperatursensor bestimmt und daraus die thermische Impedanz über die Frequenz bestimmt werden, wobei sich zischen der Heizvorrichtung und dem Temperatursensor Adsorbens befindet,
- in einem zweiten Schritt danach das Adsorbens kontrolliert mit einer der zu adsorbierenden Substanzen beladen wird und das Vorgehen des ersten Schrittes für das so beladene Adsorbens wiederholt wird,
- in weiteren Schritten danach eine schrittweise weitere Beladung wie im zweiten Schritt erfolgt, bis das Adsorbens vollständig beladen ist,
- durch Vergleich der gemessenen Impedanzen, der Wechselstrom-Widerstände, der thermischen Leitfähigkeit und der thermischen Kapazität ein Beladungskennfeld aus Wertepaaren von elektrischer und thermischer Impedanz für dieses Paar aus Adsorbens und Adsorptiv erstellt wird,
- diese Beladungskennlinie einer rechnergestützten Auswerteeinheit zugeführt wird.

12. Messverfahren nach Anspruch 11, **dadurch gekennzeichnet, dass** in weiteren Schritten der Kalibrierung vorgesehen wird, dass
- die vorangegangenen Schritte für alle weiteren in Betracht kommenden Adsorptive einzeln vorgenommen werden, wobei der Messformkörper jedes Mal zuvor entladen wird,
- die erhaltenen Beladungskennlinien als ein Beladungskennfeld zusammengeführt werden,
- dieses Beladungskennfeld einer rechnergestützten Auswerteeinheit zugeführt wird.

13. Messverfahren nach einem der Ansprüche 11 oder 12, **dadurch gekennzeichnet, dass** in weiteren Schritten der Kalibrierung vorgesehen wird, dass die Kalibrierungsschritte für verschiedene Temperaturen durchgeführt werden.

14. Verwendung eines Messverfahrens gemäß einem der Ansprüche 10 bis 13 in einem Adsorber (1), der eine Schüttung oder eine Packung aus Adsorbensmaterial enthält, **dadurch gekennzeichnet, dass** eine Vielzahl von Messformkörpern in der Schüttung oder Packung über die Lauflänge angeordnet sind.

15. Verwendung eines Messformkörpers nach einem der Ansprüche 1 bis 9 in einem Messverfahren nach einem der Ansprüche 10 bis 14, **dadurch gekennzeichnet, dass** das Adsorptiv Propan ist.

## Claims

1. Measuring body for measuring the loading status during adsorption, having at least one board (10) with devices for measuring the electrical and thermal impedance, having at least 2 electrodes (1, 2), which are capable of transmitting the current of a high-frequency alternating voltage, and at least one direct current heat resistor (6) and one temperature sensor (7), also lines for electrical connections
**characterised in that**
- the at least one board (10) is coated with an applied powder of activated carbon adsorbent (12), wherein this activated carbon adsorbent is arranged both between the electrodes (1, 2) for high-frequency alternating current and also between the heating element (6) and temperature sensor (7), and
- the activated carbon adsorbent powder (12) is fixed agglomerated in porous form onto the least one board (10).

2. Measuring body according to claim 1, **characterised in that** a planar SMD board (10) is used, in which the measuring devices of the electrical and the thermal impedance are arranged next to one another.

3. Measuring body according to claim 1, **characterised in that** two SMD boards (10a, 10b) are used, in which the measuring devices of the electrical and the thermal impedance are each arranged on a board above one another.

4. Measuring body according to any of claims 1 to 3, **characterised in that** for measuring the thermal impedance two opposite ohmic resistors are used as heating elements (6a, 6b) and a temperature sensor (7) is arranged between these two heating elements (6a, 6b).

5. Measuring body according to claim 4, **characterised in that** for measuring the thermal impedance four ohmic resistors are used as heating elements (6a, 6b, 6c, 6d), two of which (6a+6b, 6c+6d) are located opposite one another, and a temperature sensor (7) is arranged between these four heating elements (6a, 6b, 6c, 6d).

6. Measuring body according to any of claims 1 to 3, **characterised in that** for measuring the electrical impedance two opposite flat electrodes (1b, 2b) are used.

7. Measuring body according to any of claims 1 to 3, **characterised in that** for measuring the electrical impedance two concentric ring electrodes (1a, 2a) are used.

8. Measuring body according to any of claims 1 to 7, **characterised in that** at least one of the dimensions of the measuring body corresponds to that of an activated carbon adsorbent particle.

9. Measuring body according to claim 8, **characterised in that** the form and dimensions of the measuring body correspond to those of an activated carbon adsorbent particle.

10. Measuring method using a measuring body according to any of the preceding claims, **characterised in that** a calibration is carried out before the measurement to determine a measuring map.

11. Measuring method according to claim 10, **characterised in that** the calibration comprises the following steps, **in that**
- a high-frequency alternating voltage is applied firstly to a first electrode in the unloaded measuring body, the frequency of which is passed through during the measurement in a broad range, the voltage is detected at a second electrode, which is arranged opposite the first electrode and wherein adsorbent is located between the two electrodes, and thereby the drop in voltage and the current flow between the two electrodes over this frequency range and from this the curve of the electrical impedance over the frequency are determined,
- furthermore, the thermal conductivity and the thermal capacitance of the adsorbent layer are determined by applying a low-frequency temperature oscillation over a broad frequency range by means of a heating device and determining this oscillation with a temperature sensor and from this determining the thermal impedance via the frequency, wherein adsorbent is located between the heating device and the temperature sensor,
- in a second step after this the adsorbent is loaded in a controlled manner with one of the substances to be adsorbed and the procedure of the first step is repeated for the adsorbent loaded in this way,
- in further steps after this a stepwise further loading is carried out as in the second step until the adsorbent is fully loaded,
- by comparing the measured impedances, the alternating current resistances, the thermal conductivity and the thermal capacitance, a loading map is created from pairs of values of electrical and thermal impedance for this pair of adsorbent and adsorptive,
- this loading characteristics is supplied to a computer-supported evaluation unit.

12. Measuring method according to claim 11, **characterised in that** in further steps of the calibration it is provided that
- the preceding steps are carried out for all other adsorptives in consideration, whereby the measuring body is previously unloaded each time,
- the obtained characteristics are merged together into one loading map,
- this loading map is supplied to a computer-supported evaluation unit.

13. Measuring method according to any of claims 11 or 12, **characterised in that** in further steps of the calibration it is provided that the calibration steps are carried out for different temperatures.

14. Use of a measuring method according to any of claims 10 to 13 in an adsorber (1), which contains a fill or a packing of adsorbent material, **characterised in that** a plurality of measuring bodies are arranged in the fill or packing over the run length.

15. Use of a measuring body according to any of claims 1 to 9 in a measuring method according to any of claims 10 to 14, **characterised in that** the adsorptive is propane.

## Revendications

1. Corps moulé de mesure pour mesurer l'état de charge lors d'une adsorption, présentant au moins une carte (10) avec des appareils de mesure de l'impédance électrique et thermique, présentant au moins 2 électrodes (1, 2), qui sont adaptés pour transmettre le courant d'une tension alternative à hautes fréquences, ainsi qu'au moins une résistance chauffante en courant continu (6) et un capteur de température (7), également des câbles pour des raccordements électriques
**caractérisé en ce que**
- l'au moins une carte (10) est revêtue d'une poudre appliquée à partir d'un adsorbant au charbon actif (12), dans lequel ledit adsorbant au charbon actif est disposé aussi bien entre les électrodes (1, 2) pour le courant alternatif à hautes fréquences qu'également entre l'élément de chauffage (6) et le capteur de température (7), et
- la poudre adsorbante au charbon actif (12) est fixée de manière agglomérée sous la forme poreuse sur l'au moins une carte (10).

2. Corps moulé de mesure selon la revendication 1, **caractérisé en ce qu'**une carte SMD plate (10) est utilisée, dans laquelle les dispositifs de mesure de l'impédance électrique et thermique sont disposés côte à côte.

3. Corps moulé de mesure selon la revendication 1, **caractérisé en ce que** deux cartes SMD (10a, 10b) sont utilisées, dans lesquelles les dispositifs de mesure de l'impédance électrique et thermique sont disposés les uns sur les autres sur chaque carte.

4. Corps moulé de mesure selon l'une quelconque des revendications 1 à 3, **caractérisé en ce que** pour la mesure de l'impédance thermique deux résistances ohmiques opposées sont utilisées comme éléments de chauffage (6a, 6b) et un capteur de température (7) est disposé entre lesdits deux éléments de chauffage (6a, 6b).

5. Corps moulé de mesure selon la revendication 4, **caractérisé en ce que** pour la mesure de l'impédance thermique quatre résistances ohmiques sont utilisées comme éléments de chauffage (6a, 6b, 6c, 6d), parmi lesquels deux (6a+6b, 6c+6d) se font face, et un capteur de température (7) est disposé entre lesdits quatre éléments de chauffage (6a, 6b, 6c, 6d).

6. Corps moulé de mesure selon l'une quelconque des revendications 1 à 3, **caractérisé en ce que** pour la mesure de l'impédance électrique deux électrodes plates opposées (1b, 2b) sont utilisées.

7. Corps moulé de mesure selon l'une quelconque des revendications 1 à 3, **caractérisé en ce que** pour la mesure de l'impédance électrique deux électrodes annulaires concentriques (1a, 2a) sont utilisées.

8. Corps moulé de mesure selon l'une quelconque des revendications 1 à 7, **caractérisé en ce qu'**au moins l'une des dimensions du corps moulé de mesure correspond à celle d'une particule adsorbante au charbon actif.

9. Corps moulé de mesure selon la revendication 8, **caractérisé en ce que** la forme et les dimensions du corps moulé de mesure correspond à celles d'une particule adsorbante au charbon actif.

10. Procédé de mesure utilisant un corps moulé de mesure selon l'une quelconque des revendications précédentes, **caractérisé en ce qu'**un étalonnage est réalisé avant la mesure pour déterminer un champ caractéristique de mesures.

11. Procédé de mesure selon la revendication 10, **caractérisé en ce que** l'étalonnage comprend les étapes suivantes, en
- appliquant d'abord une tension alternative à hautes fréquences à une première électrode dans le corps moulé de mesure non chargé, dont la fréquence est traversée pendant la mesure dans une large plage, la tension est détectée à une seconde électrode, qui est disposée à l'opposé de la première électrode et dans lequel un adsorbant se trouve entre les deux électrodes, et ainsi la chute de tension et le flux de courant situés entre les deux électrodes sur ladite plage de fréquences et à partir de là l'évolution de l'impédance électrique sur la fréquence sont déterminés,
- en outre, la conductivité thermique et la capacité thermique de la couche adsorbante sont déterminées en appliquant une oscillation de température à basse fréquence sur une large plage de fréquences à l'aide d'un dispositif de chauffage et en déterminant ladite oscillation avec un capteur de température et à partir de là l'impédance thermique est déterminée par l'intermédiaire de la fréquence, dans lequel un adsorbant se trouve entre le dispositif de chauffage et le capteur de température,
- lors d'une seconde étape, l'adsorbant est chargé de manière contrôlée avec l'une des substances à adsorber et la procédure de la première étape est répétée pour l'adsorbant ainsi chargé,
- lors d'une autre étape, un autre chargement étape par étape s'effectue comme lors de la seconde étape jusqu'à ce que l'adsorbant soit entièrement chargé,
- en comparant les impédances mesurées, les résistances à courant alternatif, la conductivité thermique et la capacité thermique, un champ caractéristique de chargement est créé à partir de paires de valeurs de l'impédance électrique et thermique pour ladite paire d'adsorbant et d'adsorbat,
- ladite caractéristique de chargement est fournie à une unité d'évaluation assistée par ordinateur.

12. Procédé de mesure selon la revendication 11, **caractérisé en ce que** lors d'autres étapes de l'étalonnage, il est prévu que
- les étapes précédentes sont réalisées pour tous les autres adsorbats pris en considération, dans lequel le corps moulé de mesure est déchargé à chaque fois au préalable,
- les caractéristiques de chargement obtenues sont fusionnées en tant que champ caractéristique de chargement,
- ledit champ caractéristique de chargement est fourni à une unité d'évaluation assistée par ordinateur.

13. Procédé de mesure selon l'une quelconque des revendications 11 ou 12, **caractérisé en ce que** lors d'autres étapes de l'étalonnage il est prévu que les étapes d'étalonnage soient réalisées pour différentes températures.

14. Utilisation d'un procédé de mesure selon l'une quelconque des revendications 10 à 13 dans un adsorbeur (1), qui contient un lit ou une garniture de matériau adsorbant, **caractérisé en ce qu'**une pluralité de corps moulés de mesure sont disposés dans le lit ou la garniture sur la longueur.

15. Utilisation d'un corps moulé de mesure selon l'une quelconque des revendications 1 à 9 dans un procédé de mesure selon l'une quelconque des revendications 10 à 14, **caractérisé en ce que** l'adsorbat est du propane.
